# EUROPÄISCHE PATENTSCHRIFT

(11) **EP 1 607 484 B1**
(45) Veröffentlichungstag und Bekanntmachung des Hinweises auf die Patenterteilung: **02.04.2014**
(21) Anmeldenummer: 04013843.0
(22) Anmeldetag: 14.06.2004
(51) Int. Cl.: A61N 1/32, A61N 1/08, C12N 15/87, H03K 3/57

(54) **Verfahren und Schaltungsanordnung zur Behandlung von biologischem Material**
Method and circuitry for treating biological material
Méthode et circuits pour le traitement de matériel biologique

(43) Veröffentlichungstag der Anmeldung: 21.12.2005
(73) Patentinhaber: Lonza Cologne GmbH, 50829 Köln (DE)
(72) Erfinder: Müller-Hartmann, Herbert, 50937 Köln (DE)
(74) Vertreter: Remus, Alvaro Johannes

(56) Entgegenhaltungen:
- EP-B- 1 383 901
- WO-A-03/050546
- WO-A-03/076006
- US-A- 5 869 326

## Beschreibung

Die Erfindung betrifft ein Verfahren zur Behandlung von biologischem Material mittels mindestens eines durch einen ersten Spannungsimpuls erzeugten elektrischen Feldes, wobei der erste Spannungsimpuls abgebrochen wird, wenn ein vorgegebener Grenzwert für einen elektrischen Parameter über- oder unterschritten wird, sowie eine Schaltungsanordnung, insbesondere zur Durchführung des Verfahrens, mit zumindest einer Speichereinrichtung für elektrische Ladungsmengen zur Erzeugung zumindest eines Spannungsimpulses durch gezielte Entladung der Speichereinrichtung und mindestens einer Kontrolleinrichtung zur Steuerung der Entladung, mittels welcher der Spannungsimpuls bei Über- oder Unterschreiten eines vorgegebenen Grenzwertes für einen elektrischen Parameter abbrechbar ist. Die Erfindung betrifft hierbei insbesondere die Gebiete Elektroporation, Elektrofusion und Elektrostimulation von lebenden Zellen, sowie alle Anwendungen, bei denen biologisches Material einem elektrischen Feld ausgesetzt werden muss.

### Hintergrund

Das Einbringen biologisch aktiver Moleküle, wie beispielsweise DNA, RNA oder Proteine, in lebende Zellen stellt ein wichtiges Instrument zur Untersuchung biologischer Funktionen dieser Moleküle dar. Eine bevorzugte Methode zum Einbringen von Fremdmolekülen in die Zellen ist dabei die Elektroporation, welche im Gegensatz zu chemischen Methoden nicht auf den gleichzeitigen Transport anderer biologisch aktiver Moleküle angewiesen ist. Bei der Elektroporation werden die Fremdmoleküle aus einer an die Zellen angepassten Pufferlösung oder einem Zellkulturmedium durch einen kurzzeitigen Stromfluss in die Zellen eingebracht, wobei durch die Wirkung der kurzen elektrischen Spannungsimpulse bzw. des dadurch entstehenden elektrischen Feldes die Zellmembran für die Fremdmoleküle durchlässig gemacht wird. Die Zellsuspension befindet sich dabei häufig in einer sogenannten Küvette, d.h. einem schmalen, nach oben offenen Gefäß, das in der Nähe seines Bodens zwei gegenüberliegende, parallele Elektroden in den Seitenwänden aufweist, welche zum Anlegen der elektrischen Spannung dienen. Durch die kurzzeitig entstehenden "Poren" in der Zellmembran gelangen die biologisch aktiven Moleküle zunächst in das Zytoplasma, in dem sie ggf. bereits ihre zu untersuchende Funktion ausüben können, und daraufhin unter bestimmten Bedingungen auch in den Zellkern.

Durch das kurzzeitige Anlegen eines starken elektrischen Feldes, d.h. eines kurzen Spannungsimpulses mit hoher Stromdichte, können darüber hinaus auch Zellen, Zellderivate, subzelluläre Partikel und/oder Vesikel fusioniert werden. Bei dieser sogenannten Elektrofusion werden die Zellen beispielsweise zunächst durch ein inhomogenes elektrisches Wechselfeld in engen Membrankontakt gebracht. Durch das anschließende Anlegen eines elektrischen Feldpulses kommt es dann zur Interaktion von Membranteilen, die schließlich zur Fusion führt. Für die Elektrofusion können dabei vergleichbare apparative Vorrichtungen verwendet werden, wie für die Elektroporation. Darüber hinaus können lebende Zellen durch elektrische Felder auch in einer ihre Eigenschaften verändernden Weise stimuliert werden.

Wenn beim Aufbau eines elektrischen Feldes mit einer Feldstärke von mehreren hundert Volt pro Zentimeter in einer wässrigen Lösung der elektrische Widerstand in einem sehr kurzen Zeitabschnitt, beispielsweise unter 1 µs, zusammen bricht und somit der Strom sehr schnell und stark ansteigt, dann kann eine sogenannte Blitzentladung erfolgen. Bei einer Blitzentladung kommt es durch den kurzzeitigen Leistungs- bzw. Wärmeanstieg einerseits zu physikalischen Begleiterscheinungen wie Blitzen, Knallen und Spritzen der Lösung und andererseits zu einer irreversiblen Schädigung oder dem Tod der Zellen. Eine Blitzentladung gefährdet daher in der Regel nicht nur die Sicherheit der umstehenden Personen und der Gerätschaften, sondern führt auch zu einem Verlust des eingesetzten biologischen Materials.

### Stand der Technik

Aus der WO 02/086129 A1 sind eine Schaltungsanordnung zur Einbringung von biologisch aktiven Molekülen in den Zellkern eukaryontischer Zellen mittels elektrischen Stroms, oder zur Behandlung von Zellen, Zellderivaten, subzellulären Partikeln und/oder Vesikeln mit elektrischem Strom, sowie ein entsprechendes Verfahren bekannt. Die Schaltungsanordnung besteht aus zwei Speichereinrichtungen für elektrische Ladungsmengen, welche von je einem Hochspannungsnetzteil gespeist werden. Die Speichereinrichtungen sind mit jeweils einem Leistungshalbleiter zur Übertragung der in den Speichereinrichtungen vorhandenen Ladungsmengen auf eine Zellsuspension verbunden. Die Leistungshalbleiter werden mittels einer Kontrolleinrichtung ansteuert bzw. geschaltet. Bei dieser bekannten Schaltungsanordnung ist ferner vorgesehen, dass zumindest ein erster Spannungsimpuls mit der Kondensatorspannung der Speichereinrichtung durch Ansteuern eines Leistungshalbleiters für einen voreingestellten Zeitraum (T₁) auf die Zellsuspension übertragbar ist. Zur weiteren Sicherheit des Benutzers und der verwendeten Proben ist vorgesehen, dass mittels einer Überstromschaltstufe eine Überstromabschaltung zumindest für den ersten Spannungsimpuls vorgesehen ist, durch die der jeweilige Impuls abgebrochen wird. Die Überstromabschaltung ermöglicht somit die Unterbrechung des Spannungsimpulses für den Fall, dass voreingestellte Grenzwerte überschritten werden. Wenn also beispielsweise beim Aufbau eines elektrischen Feldes der Strom zu stark ansteigt, dann kann eine Blitzentladung und damit eine Schädigung der Zellen durch den Abbruch des Spannungsimpulses vermieden werden. In Abhängigkeit vom Zeitpunkt des Abbruchs hat dies aber den Nachteil, dass der Erfolg der Behandlung nicht eintritt, also beispielsweise die Transfektionseffizienz zu gering ist. Wird der Spannungsimpuls zu einem sehr frühen Zeitpunkt abgebrochen, muss der entsprechende Reaktionsansatz daher trotz des Erhalts der Lebensfähigkeit der Zellen verworfen werden oder ist jedenfalls nur in sehr geringem Maße brauchbar.

Der Erfindung liegt daher die Aufgabe zugrunde, ein Verfahren und eine Schaltungsanordnung der eingangs genannten Art zu schaffen, welche eine erfolgreiche Behandlung von biologischem Material auch dann ermöglichen, wenn der erste Spannungsimpuls abgebrochen wurde.

### Beschreibung der Erfindung

Erfindungsgemäß ist zur Lösung der Aufgabe für das Verfahren vorgesehen, dass der erste Spannungsimpuls nach dem Abbruch durch zumindest einen weiteren Spannungsimpuls fortgesetzt wird. Überraschender Weise hat sich gezeigt, dass der Erfolg der Behandlung der Zellen doch noch sichergestellt werden kann, wenn man den abgebrochenen Spannungsimpuls fortsetzt. Durch den weiteren Spannungsimpuls werden die Zellen nochmals einem elektrischen Feld ausgesetzt, welches vorzugsweise dem durch den ersten Spannungsimpuls erzeugten entspricht, so dass die abgebrochene Behandlung der Zellen fortgesetzt werden und der gewünschte Erfolg doch noch eintreten kann. So kann durch das erfindungsgemäße Verfahren beispielsweise bei einer Elektroporation die Transfektionseffizienz bei der Transfektion von eukaryontischen Zellen mit Nukleinsäuren durch das Fortsetzen bzw. Wiederholen des Spannungsimpulses nach einer Blitzentladung deutlich verbessert werden. Der Vorteil des erfindungsgemäßen Verfahrens liegt also darin, dass aufgrund des zufällig auftretenden Abbruchs eines Spannungsimpulses auftretende nicht vorhersagbare und unreproduzierbare Ergebnisse vermieden bzw. ausgeglichen werden. Als elektrische Parameter, welche den Abbruch des Spannungsimpulses bewirken können, kommen beispielsweise eine Steigung eines Spannungsabfalls (Flanke), ein Zusammenbruch des Widerstandes, die Stromstärke oder die Steigung des Stromanstiegs (Flanke) in Frage. Erfindungsgemäß erfolgt das Fortsetzen des Spannungsimpulses dadurch, dass für den ersten Spannungsimpuls eine bestimmte Dauer T1 vorgegeben wird und die Dauer T2 des weiteren Spannungsimpulses zumindest gleich der Dauer T1 abzüglich des zwischen dem Beginn des ersten Spannungsimpulses und dem Abbrechen desselben liegenden Zeitraums Tx ist. Auf diese Weise wird sichergestellt, dass die Zellen in der Summe zumindest für die gleiche Zeit dem elektrischen Feld ausgesetzt werden, wie für den ersten Spannungsimpuls vorgesehen. Wird also beispielsweise für den ersten Spannungsimpuls eine Dauer T1 = 500 µs voreingestellt, dieser aber aufgrund einer drohenden Blitzentladung nach einem Zeitraum von Tx = 100 µs abgebrochen, so wird die Dauer T2 des weiteren Spannungsimpulses vorteilhafterweise aus T2 = T1 - Tx berechnet, d.h. für T2 ergibt sich eine Dauer von 400 µs. Der weitere Spannungsimpuls setzt also den ersten Spannungsimpuls in der Weise fort, dass die Zellen dem elektrischen Feld in der Summe für die ursprünglich eingestellte bzw. vorgegebene Dauer von 500 µs ausgesetzt sind. Auf diese Weise wird einerseits vermieden, dass die Zellen durch eine zu lange Behandlung geschädigt werden, und andererseits sichergestellt, dass die Ergebnisse reproduzierbar sind. Alternativ kann die Dauer T2 auch länger sein als die Dauer T1 abzüglich des zwischen dem Beginn des ersten Spannungsimpulses und dem Abbrechen desselben liegenden Zeitraums Tx, d.h. T2 > T1 - Tx. Bei einer Dauer T1 = 500 µs und einem Zeitraum bis zum Abbruch von Tx = 100 µs kann als Dauer für den weiteren Spannungsimpuls auch beispielsweise eine Dauer T2 = 600 µs gewählt werden. Hierdurch können mögliche Verluste bzw. Nachteile durch die Pausenzeit zwischen dem Abbruch des ersten Spannungsimpulses und dem Auslösen des weiteren Spannungsimpulses ausgeglichen werden, was sich ebenfalls positiv auf die erzielten Ergebnisse auswirkt.

Als T1 kann beispielsweise eine Dauer aus einem Intervall von 10 µs bis 1 ms ausgewählt werden.

In weiterer vorteilhafter Ausgestaltung der Erfindung ist vorgesehen, dass als Feldstärke des weiteren Spannungsimpulses die gleiche Feldstärke wie für den ersten Spannungsimpuls vorgegeben wird. Hierdurch ist sichergestellt, dass die Zellen unter konstanten Bedingungen behandelt werden und der (die) weitere(n) Spannungsimpuls(e) eine Fortsetzung des ersten Spannungsimpulses darstellt bzw. darstellen. Dies wirkt sich auch positiv auf die Reproduzierbarkeit der Ergebnisse aus.

Vorzugsweise wird in Abhängigkeit von der Anwendung eine Feldstärke von 2 bis 10 KV/cm vorgegeben. Es können aber für besondere Anwendungen oder Zelltypen auch geringere oder höhere Feldstärken eingestellt werden.

In einer besonderen Ausführungsform der Erfindung ist vorgesehen, dass zwischen dem Abbrechen des ersten Spannungsimpulses und der Erzeugung des weiteren Spannungsimpulses eine bestimmte Pausenzeit vorgegeben wird, vorzugsweise ein Zeitraum von mindestens 40 µs, besonders bevorzugt 50 bis 600 µs, hierbei insbesondere 100 µs. Durch das definierte Einstellen der Pausenzeit kann das erfindungsgemäße Verfahren an die Art der Anwendung, das erwünschte Ziel und/oder den Zelltyp angepasst werden, so dass eine Optimierung der Ergebnisse möglich ist. Dabei ist es allgemein besonders vorteilhaft, wenn die Pausenzeit nicht kürzer als 40 µs ist, damit sich einerseits die Bedingungen innerhalb des Reaktionsansatzes nach dem kurzzeitigen Stromanstieg und dem Abbruchereignis normalisieren können und andererseits die Zellen eine kurze "Erholungsphase" haben.

In vorteilhafter Ausgestaltung der Erfindung ist ferner vorgesehen, dass insgesamt mindestens zwei weitere Spannungsimpulse erzeugt werden, wenn die vorhergegangenen weiteren Spannungsimpulse abgebrochen werden. Bei dieser Ausführungsform wird die Möglichkeit berücksichtigt, dass auch der weitere Spannungsimpuls oder mehrere der weiteren Spannungsimpulse aufgrund einer Über- oder Unterschreitung eines Grenzwertes für einen elektrischen Parameter abgebrochen werden können. Durch die Vorgabe der Möglichkeit mehrerer Wiederholungsversuche wird das erfindungsgemäße Verfahren weiter verbessert, da die Wahrscheinlichkeit eines endgültig fehlerhaften Versuchs bzw. einer unvollständigen Behandlung deutlich reduziert werden kann. Vorzugsweise wird dabei die Möglichkeit der Erzeugung von 2 oder 3 weiteren Spannungsimpulsen vorgegeben bzw. eingestellt. Nach Abbruch des weiteren Spannungsimpulses kann also ein zusätzlicher weiterer Spannungsimpuls ausgelöst werden (2 weitere Spannungsimpulse, n = 2). Wird auch der letztere abgebrochen, so wird ein dritter weiterer Spannungsimpuls erzeugt (3 weitere Spannungsimpulse, n = 3). Hierbei handelt es sich allerdings um die vorgegebene Möglichkeit zusätzlicher weiterer Spannungsimpulse. Wird dagegen die vorgegebene Dauer T1 insgesamt erreicht, so werden keine weiteren Spannungsimpulse mehr ausgelöst.

Für den Fall, dass mehrere weitere Spannungsimpulse möglich sind und für den ersten Spannungsimpuls eine bestimmte Dauer T1 vorgegeben wird, ist es vorteilhaft, wenn die Gesamtdauer Ts der weiteren Spannungsimpulse zumindest gleich der Dauer T1 abzüglich des zwischen dem Beginn des ersten Spannungsimpulses und dem Abbrechen desselben liegenden Zeitraums Tx ist. Auf diese Weise wird auch bei dieser Ausführungsform sichergestellt, dass die Zellen in der Summe zumindest für die gleiche Zeit dem elektrischen Feld ausgesetzt werden, wie es für den ersten Spannungsimpuls vorgesehen war. Wird also beispielsweise für den ersten Spannungsimpuls eine Dauer T1 = 800 µs voreingestellt, dieser aber aufgrund einer drohenden Blitzentladung nach einem Zeitraum von Tx = 250 µs abgebrochen, so wird die Dauer T2 des weiteren Spannungsimpulses aus T2 = T1 - Tx berechnet, d.h. für T2 ergibt sich eine Dauer von 550 µs. Wird aber nun auch der weitere Spannungsimpuls nach einem Zeitraum Ty = 350 µs abgebrochen, so ergibt sich für als Dauer T3 für den zusätzlichen weiteren Spannungsimpuls eine Dauer von T3 = T1 - (Tx + Ty) oder T3 = T2 - Ty, d.h. also T3 = 200 µs. Die Summe Ts = Ty + T3 (= T1 - Tx) ergibt also 550 µs, so dass die Zellen insgesamt für 800 µs (= T1) einem elektrischen Feld ausgesetzt sind. Die weiteren Spannungsimpulse setzen also den ersten Spannungsimpuls in der Weise fort, dass die Zellen dem elektrischen Feld in der Summe für die ursprünglich eingestellte bzw. vorgegebene Dauer ausgesetzt sind (T1 = Tx + Ty + ... + Tn). Auf diese Weise wird auch bei dieser Ausführungsform einerseits vermieden, dass die Zellen durch eine zu lange Behandlung geschädigt werden, und andererseits sichergestellt, dass die Ergebnisse reproduzierbar sind. Alternativ kann die Dauer Ts auch länger sein als die Dauer T1 abzüglich des zwischen dem Beginn des ersten Spannungsimpulses und dem Abbrechen desselben liegenden Zeitraums Tx, d.h. Ts > T1 - Tx. Bei einer Dauer T1 = 800 µs und einem Zeitraum bis zum Abbruch des ersten Spannungsimpulses von Tx = 250 µs und einem Zeitraum bis zum Abbruch des weiteren Spannungsimpulses Ty = 350 µs kann als Dauer für den zusätzlichen weiteren Spannungsimpuls auch beispielsweise eine Dauer T3 = 300 µs gewählt werden. Hierdurch können mögliche Verluste bzw. Nachteile durch die Pausenzeiten zwischen den Abbrüchen der Spannungsimpulse und dem Auslösen der weiteren Spannungsimpulse ausgeglichen werden, was sich ebenfalls positiv auf die erzielten Ergebnisse auswirkt.

Die Aufgabe wird ferner hinsichtlich der Schaltungsanordnung dadurch gelöst, dass zumindest eine Steuereinrichtung zur Erfassung des zeitlichen Verlaufs des Spannungsimpulses vorgesehen ist, durch welche eine Fortsetzung der Entladung nach dem Abbruch steuerbar ist. Durch das Einführen einer zusätzlichen Steuereinrichtung in die Schaltungsanordnung, die den zeitlichen Verlauf des ersten (und ggf. der weiteren) Spannungsimpulse(s) erfasst, ist es möglich, die verbleibende Dauer des weiteren Spannungsimpulses bzw. der weiteren Spannungsimpulse (T2 = T1 - Tx bzw. T3 = T1 - (Tx + Ty) usw.), in der nach dem Abbruch keine Spannung mehr anlag, zu berechnen. Nach einer vorgegebenen bzw. zu programmierenden Pausenzeit kann die Steuereinrichtung die Entladung der Speichereinrichtung derart steuern, dass diese fortgesetzt und somit die Entladungszeit komplettiert wird. Die Steuereinheit ist also derart ausgebildet, dass sie den Zeitraum vom Beginn des ersten Spannungsimpulses und ggf. der weiteren Spannungsimpulse bis zum Abbruch desselben erfasst und die noch verbleibende Dauer des weiteren Spannungsimpulses (T2 = T1 - Tx bzw. T3 = T1 - (Tx + Ty) usw.) berechnet. Auf diese Weise kann die Entladung der Speichereinrichtung durch die Steuereinrichtung derart gesteuert werden, dass der erste Spannungsimpuls fortgesetzt bzw. komplettiert werden kann, so dass die Behandlung des biologischen Materials trotz des erfolgten Abbruchs eines oder mehrerer Spannungsimpulse erfolgreich und reproduzierbar durchgeführt werden kann.

In einer besonderen Ausführungsform der Erfindung ist vorgesehen, dass die Steuereinrichtung ein analoges signalverarbeitendes Bauteil, beispielsweise ein Kondensator, ist. Hierbei übernimmt beispielsweise ein Kondensator die Aufgabe, die Zeit zu integrieren, in der Spannung anliegt. Der Kondensator wird nur geladen, solange Spannung anliegt. Eine Hardware-bestimmte Zeitschaltung erlaubt es dann, den Schaltkreis wieder zu schließen, solange der Kondensator noch nicht voll ist oder einen Schwellenwert nicht erreicht hat.

In einer bevorzugten Ausführungsform ist vorgesehen, dass die Steuereinrichtung ein digitales signalverarbeitendes Bauteil, beispielsweise ein DSP, ist. Durch ein DSP- (Digital Signal Processing) Bauteil, das beispielsweise eine Schaltvorrichtung kontrolliert, ist es möglich, den Zeitverlauf des ersten Spannungsimpulses bzw. der weiteren Spannungsimpulse zu verfolgen. Wenn es zum Abbruch des Spannungsimpulses durch eine Kontrolleinrichtung kommt, wird dies vom DSP-Bauteil erkannt. Das DSP-Bauteil errechnet die verbleibende Zeit, in der keine mehr Spannung anlag. Nach einer zu programmierenden Pausenzeit kann das DSP die Schaltvorrichtung dann derart steuern, dass die Entladung der Speichereinrichtung fortgesetzt wird, beispielsweise durch entsprechende Steuerung der Kontrolleinrichtung.

In einer bevorzugten Ausführungsform der erfindungsgemäßen Schaltungsanordnung kann die Steuereinrichtung und/oder die Kontrolleinrichtung mit einer Schaltvorrichtung verbunden sein. Diese Schaltvorrichtung ist, vorzugsweise über eine Potentialtrennstufe, mit einem Spannungsschalter verbunden. Die Speichereinrichtung ist mit einem Leistungshalbleiter verbunden, über den die Entladung der Speichereinrichtung erfolgt. Wenn der Spannungsschalter ebenfalls mit dem Leistungshalbleiter verbunden ist, kann die Entladung der Speichereinrichtung folglich durch die Steuereinrichtung und/oder die Kontrolleinrichtung gesteuert werden. Dadurch, dass die Steuereinrichtung mit der Schaltvorrichtung verbunden ist, kann diese den zeitlichen Verlauf der Entladung durch eine Kontrolle und Erfassung des Schaltzustandes des Schaltvorrichtung erfassen und somit die Dauer des jeweiligen Spannungsimpulses bestimmen.

Die Schaltvorrichtung ist in vorteilhafter Ausgestaltung der Erfindung durch die Kontrolleinrichtung schaltbar, d.h. die Kontrolleinrichtung kann die Schaltvorrichtung öffnen und schließen, so dass letztendlich der mit der Speichereinrichtung verbundene Leistungshalbleiter geöffnet oder geschlossen und somit die Entladung der Speichereinrichtung gesteuert wird. Dabei kann die Kontrolleinrichtung eine Abschaltvorrichtung enthalten, welche die Entladung abbricht, wenn ein vorgegebener Grenzwert für einen elektrischen Parameter über- oder unterschritten wird. Als elektrische Parameter können dabei die Steigung eines Spannungsabfalls (Flanke), der Zusammenbruch des Widerstandes, die Stromstärke oder die Steigung des Stromanstiegs (Flanke) verwendet werden. Wird also ein vorgegebener Grenzwert für einen dieser Parameter über- oder unterschritten, so schaltet die Abschaltvorrichtung die Schaltvorrichtung derart, dass die Entladung der Speichereinrichtung abgebrochen wird. Beispielsweise kann bei einer drohenden Blitzentladung die Steigung des Anstiegs der Stromstärke in einem sehr kurzen Zeitintervall gemessen werden und bei Überschreiten eines festgelegten Grenzwertes der Spannungsimpuls durch die Kontrolleinrichtung bzw. die Abschaltvorrichtung abgebrochen werden (Flankenabschaltung).

Die Schaltvorrichtung kann auch unmittelbar durch die Steuereinrichtung schaltbar sein, so dass diese die Entladung der Speichereinrichtung durch Schalten der Schaltvorrichtung steuern kann. Die Steuereinrichtung kann also über die Schaltvorrichtung nicht nur den zeitlichen Verlauf des jeweiligen Spannungsimpulses erfassen, sondern auch die weiteren Spannungsimpulse auslösen. Vorzugsweise erfolgt die Steuerung der Entladung durch die Steuereinrichtung aber über die Kontrolleinrichtung.

Die Erfindung umfasst ferner ein Programmelement, das mittels einer elektronischen Datenverarbeitungseinrichtung lesbar und ausführbar ist und das, wenn es ausgeführt wird, dazu geeignet ist, das erfindungsgemäße Verfahren durchzuführen, sowie ein Programmelement, das mittels einer elektronischen Datenverarbeitungseinrichtung lesbar und ausführbar ist und das, wenn es ausgeführt wird, dazu geeignet ist, die erfindungsgemäße Schaltungsanordnung zu steuern. Insgesamt umfasst die Erfindung also auch Computerprogramme, welche das erfindungsgemäße Verfahren und/oder die erfindungsgemäße Schaltungsanordnung steuern. Die Programmelemente sind dabei vorzugsweise in einer Speichereinheit einer Vorrichtung (Elektroporator) abgelegt, in der auch die erfindungsgemäße Schaltungsanordnung angeordnet ist. Ein geeigneter Prozessor kann dabei auf die Programmelemente zugreifen und diese verarbeiten bzw. ausführen.

Die Erfindung umfasst auch jedes Speichermedium, das mittels einer elektronischen Datenverarbeitungseinrichtung lesbar ist und auf dem eines oder beide der genannten Programmelemente gespeichert ist oder sind.

Das erfindungsgemäße Verfahren und die erfindungsgemäße Schaltungsanordnung sind in vorteilhafter Weise zur Transfektion ruhender oder teilungsaktiver eukaryontischer Zellen einsetzbar bzw. geeignet. Ebenso sind diese zur Transfektion von primären Zellen wie Zellen des menschlichen Bluts, pluripotenter Vorläuferzellen des menschlichen Bluts, primärer menschlicher Fibroblasten, Endothelzellen, Muskelzellen oder Melanozyten geeignet und können zu analytischen oder diagnostischen Zwecken oder zur Herstellung eines Arzneimittels zur ex-vivo-Gentherapie eingesetzt werden.

Das erfindungsgemäße Verfahren und die erfindungsgemäße Schaltungsanordnung eignen sich darüber hinaus beispielsweise auch für die Elektrofusion, d.h. Verfahren zur Fusion von Zellen, Zellderivaten, subzellulären Partikeln und/oder Vesikeln mittels elektrischen Stroms, bei dem beispielsweise die Zellen, Zellderivate, subzellulären Partikel und/oder Vesikel zunächst in zweckmäßiger Dichte in einer wässrigen Lösung suspendiert werden, die Suspension anschließend in eine Küvette überführt wird, und schließlich eine elektrische Spannung an die Elektroden der Küvette angelegt und ein Stromfluss durch die Suspension erzeugt wird. Alternativ können beispielsweise auch adhärente Zellen, Zellderivate, subzelluläre Partikel und/oder Vesikel oder aber auch beispielsweise adhärente Zellen mit suspendierten Zellen, Zellderivaten, subzellulären Partikeln oder Vesikeln fusioniert werden.

Der Begriff "biologisches Material" umfasst Zellen, Zellderivate, subzelluläre Partikel und Vesikel, sowie Nukleinsäuren, Peptide, Proteine, Polysaccharide, Lipide oder Kombinationen oder Derivate dieser Moleküle.

Der Begriff "biologisch aktive Moleküle" umfasst Nukleinsäuren, Peptide, Proteine, Polysaccharide, Lipide oder Kombinationen oder Derivate dieser Moleküle, solange sie in Zellen, Zellderivaten, subzellulären Partikeln oder Vesikeln eine biologische Aktivität entfalten.

Geeignete Behältnisse zur Aufnahme des biologischen Materials bzw. der Reaktionsansätze sind beispielsweise Küvetten mit einem Elektrodenabstand von 2 mm oder 1 mm, z. B. handelsübliche Küvetten für die Elektroporation.

Die Erfindung wird im weiteren durch die folgenden Figuren beispielhaft näher erläutert.

Es zeigt
- Figur 1: ein Blockschaltbild einer erfindungsgemäßen Schaltungsanordnung,
- Figur 2: ein Blockschaltbild einer besonderen Ausführungsform einer erfindungsgemäßen Schaltungsanordnung,
- Figur 3: ein Flussdiagramm zur Erläuterung des erfindungsgemäßen Verfahrens,
- Figur 4: ein Flussdiagramm zur Erläuterung einer besonderen Ausführungsform des erfindungsgemäßen Verfahrens und
- Figur 5: Diagramme experimenteller Daten für künstlich unterbrochene Spannungsimpulse.

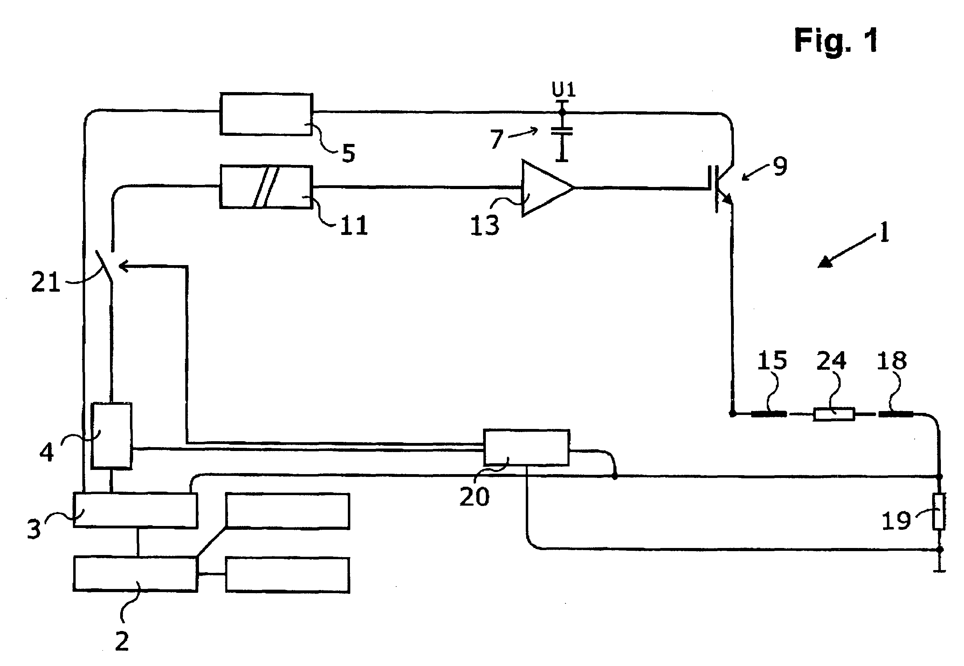

**Figur 1** zeigt ein Blockschaltbild einer erfindungsgemäßen Schaltungsanordnung 1 mit einer Einstellungseinheit 2 zur Eingabe vorzugebender Parameter, einer zentralen Steuerungseinheit 3 zur Steuerung der Schaltungsanordnung 1 sowie einem Hochspannungsnetzteil 5. Das Hochspannungsnetzteil 5 speist eine nachgeschaltete Speichereinrichtung 7, die beispielsweise ein Kondensator oder eine Gruppe von Kondensatoren sein kann, welche mit einer Spannung U1 aufladbar ist. Die Speichereinrichtung 7 ist mit einem zur Abgabe eines Spannungsimpulses (U1) bzw. zur Entladung der Speichereinrichtung 7 vorgesehenen Leistungshalbleiter 9 verbunden. Der Leistungshalbleiter 9 ist, hier über eine Potentialtrennstufe 11, mittels eines Spannungsschalters 13 durch die Steuerungseinheit 3, eine Kontrolleinrichtung 20 und eine Steuereinrichtung 4 ansteuerbar. Die Speichereinrichtung 7 ist mit dem Eingang des Leistungshalbleiters 9 unmittelbar verbunden, wobei der Leistungshalbleiter 9 beispielsweise aus einem IGBT bestehen kann. Der Begriff "Leistungshalbleiter" soll jedoch auch sämtliche andere elektronische Bauelemente oder Bauelementanordnungen umfassen, mittels derer zu schaltende Spannungen und Ströme mit den erforderlichen Schaltzeiten geschaltet werden können. Der Ausgang des Leistungshalbleiters 9 ist unmittelbar mit einem Küvettenanschluss 15 verbunden. Bei der Küvette 24 handelt es sich um ein Reaktionsgefäß, das der Aufnahme einer wässrigen Lösung und des zu behandelnden biologischen Materials dient und in dem das elektrische Feld erzeugt wird. Ein zweiter Küvettenanschluss 18 ist über einen Widerstand 19 mit Masse verbunden. Bei dem Widerstand 19 handelt es sich um ein Mess-Shunt, um beispielsweise den Spannungsabfall zu messen und der Kontrolleinrichtung 20 zuzuführen. Die Kontrolleinrichtung 20 kann über eine Schaltvorrichtung 21 eine Unterbrechung des Spannungsimpulses über die Potentialtrennstufe 11 und den Spannungsschalter 13 bewirken. Wird beispielsweise ein vorgegebener Grenzwert für einen elektrischen Parameter über- oder unterschritten, so schaltet eine in der Kontrolleinrichtung 20 angeordnete, hier nicht dargestellte Abschaltvorrichtung die Schaltvorrichtung 21 derart, dass die Entladung der Speichereinrichtung 7 abgebrochen wird. Beispielsweise kann bei einer drohenden Blitzentladung die Steigung des Anstiegs der Stromstärke in einem sehr kurzen Zeitintervall gemessen werden und bei Überschreiten eines festgelegten Grenzwertes der Spannungsimpuls durch die Kontrolleinrichtung 20 bzw. die Abschaltvorrichtung abgebrochen werden (Flankenabschaltung). Erfindungsgemäß ist die Schaltvorrichtung 21 durch die Steuereinrichtung 4 schaltbar, so dass diese die Entladung der Speichereinrichtung 7 durch Schalten der Schaltvorrichtung 21 steuern kann. Die Steuereinrichtung 4 kann also über die Schaltvorrichtung 21 einerseits den zeitlichen Verlauf des jeweiligen Spannungsimpulses, d.h. den Zeitpunkt eines Abbruchs des Impulses, erfassen und die Dauer des weiteren Spannungsimpulses berechnen, und andererseits die weiteren Spannungsimpulse durch Ansteuern der Schaltvorrichtung 21 auslösen. Dabei erfolgt die Steuerung der Entladung durch die Steuereinrichtung 4 vorzugsweise mittelbar über die Kontrolleinrichtung 20. Die Steuereinrichtung 4 kann die Schaltvorrichtung 21 aber alternativ auch direkt ansteuern. Im Fall der Kontrolleinrichtung 20 liegt der niederohmige Messwiderstand 19 hinter den Küvettenanschlüssen 15,18 und ist gegen Masse verschaltet, sodass die Übertragung von Hochspannungsimpulsen ausgeschlossen werden kann. Die Speichereinrichtung 7 kann mehrere Kondensatoren der notwendigen Kapazität und Durchschlagsspannung umfassen, so dass eine entsprechend hohe Ladungsmenge gespeichert und auf den Küvettenanschluss 15 übertragen werden kann.

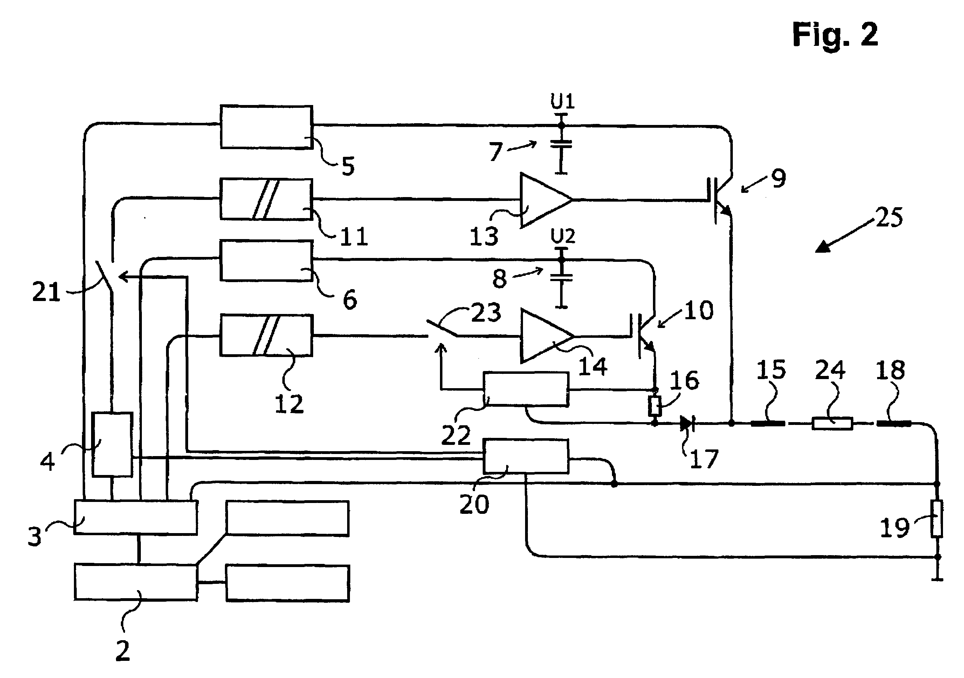

**Figur 2** zeigt ein Blockschaltbild einer besonderen Ausführungsform einer erfindungsgemäßen Schaltungsanordnung 25, die im wesentlichen der Schaltungsanordnung 1 gemäß Figur 1 entspricht, jedoch im Unterschied dazu eine weitere Speichereinrichtung 8, die mit einer Spannung U2 aufladbar ist, zur Abgabe eines zweiten Spannungsimpulses aufweist. Diese Schaltungsanordnung 25 ist daher für Anwendungen geeignet, bei denen zwei kurz aufeinander folgende Spannungsimpulse abgegeben werden müssen. So kann es beispielsweise für die Transfektion bestimmter Zelltypen vorteilhaft sein, zunächst einen kurzen Spannungsimpuls mit hoher Feldstärke und unmittelbar darauf folgend einen, vorzugsweise ohne Unterbrechung auf den ersten Spannungsimpuls folgenden, zweiten Spannungsimpuls von längerer Dauer, aber mit geringerer Feldstärke, zu erzeugen. Zwei Hochspannungsnetzteile 5,6 speisen zu diesem Zweck jeweils eine nachgeschaltete Speichereinrichtung 7,8, die jeweils beispielsweise ein Kondensator oder eine Gruppe von Kondensatoren sein können, welche mit jeweils einem zur Abgabe eines Spannungsimpulses bzw. zur Entladung der Speichereinrichtungen 7,8 vorgesehenen Leistungshalbleiter 9,10 verbunden sind. Der Leistungshalbleiter 10 ist, hier über die Potentialtrennstufe 12, mittels eines Spannungsschalters 14 durch die Steuerungseinheit 3, eine Kontrolleinrichtung 22 und die Steuereinrichtung 4 ansteuerbar. Die Speichereinrichtungen 7,8 sind mit den Eingängen der Leistungshalbleiter 9,10 unmittelbar verbunden, wobei die Speichereinrichtungen 7,8 je nach verwendeter Feldstärke und Impulsdauer aus einem oder mehreren Kondensatoren bestehen können. Der Leistungshalbleiter 9 kann beispielsweise aus einem IGBT und der Leistungshalbleiter 10 aus einem MOSFET bestehen. Bevorzugt bestehen jedoch beide Leistungshalbleiter 9,10 aus einem IGBT. Der Ausgang des Leistungshalbleiters 10 ist über einen Widerstand 16 und eine Diode 17 mit dem Küvettenanschluss 15 verbunden, so dass kein Impuls über den zweiten Leistungshalbleiter 10 zurückfließen kann, falls beide Leistungshalbleiter 9, 10 gleichzeitig angesteuert sind. Die Diode 17 ist hierzu kathodenseitig mit dem Küvettenanschluss 15 verbunden. Eine zweite Kontrolleinrichtung 22 kann über einen Schalter 23 eine Ansteuerung des Spannungsschalters 14 für den Leistungshalbleiter 10 unterbrechen. Der Kontrolleinrichtung 22 wird die über den Widerstand 16 anliegende Spannung zugeleitet, um im Fall einer Überschreitung eines Maximalstroms eine Stromabschaltung herbeizuführen. Dadurch, dass der Widerstand 16 unmittelbar im Hochspannungskreis liegend angeordnet ist, befindet sich der Schalter 23 hinter der Potentialtrennstufe 12, sodass keine Hochspannungsimpulse in die Steuerungseinheit 3 gelangen können und somit das Bedienungspersonal nicht gefährdet ist. Je nach Verwendungszweck der Schaltungsanordnung 25 können ein oder mehrere Hochspannungsnetzteile 5,6 mit zugehörigen Speichereinrichtungen 7,8 sowie notwendigen Potentialtrennstufen 11,12 und Spannungsschaltern 13,14 zur Ansteuerung der Leistungshalbleiter 9,10 eingesetzt werden. Die Speichereinrichtungen 7,8 sind dabei jeweils mit einem oder mehreren Kondensatoren der notwendigen Kapazität und Durchschlagsspannung ausgestattet, sodass eine entsprechend hohe Ladungsmenge gespeichert und auf dem Küvettenanschluss 15 übertragen werden kann. Hinsichtlich der Steuerung der Entladung der Speichereinrichtung 7 durch die Steuereinrichtung 4 entspricht die Schaltungsanordnung 25 exakt der Schaltungsanordnung 1 gemäß Figur 1.

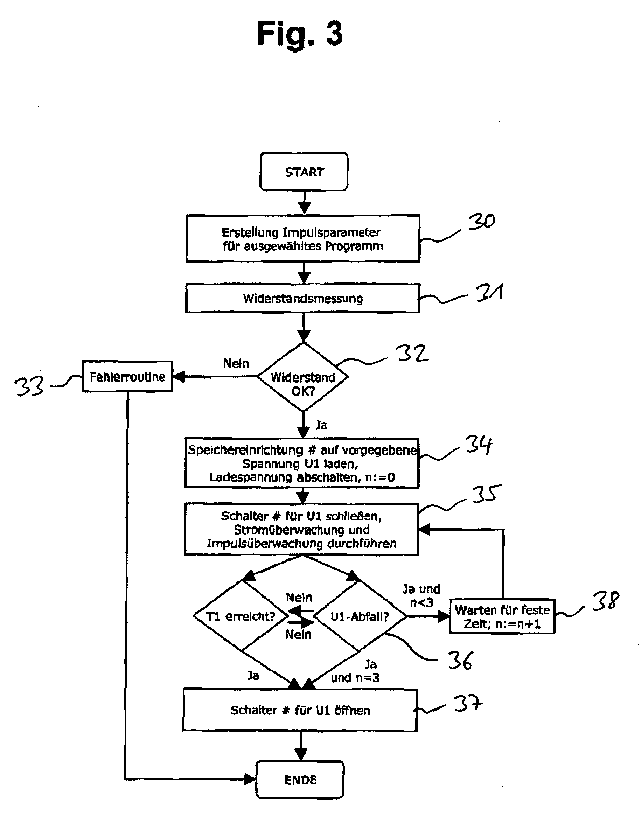

**Figur 3** zeigt ein schematisches Flussdiagramm des Verfahrens gemäß einer bevorzugten Ausführungsform der Erfindung. Zunächst werden die erforderlichen Impulsparameter (z.B. Impulsdauer T1 und Feldstärke) manuell über die Einstellungseinheit eingegeben oder durch Auslesen aus einer Speicherkarte eingestellt (hier nicht dargestellt). Nach dem Start der Routine (z.B. durch Betätigung einer entsprechenden Auslösetaste) werden in Schritt 30 zunächst die Impulsparameter für das ausgewählte Programm erstellt. In Schritt 31 wird dann der ohmsche Widerstand der Küvette durch kurzzeitiges Anlegen einer Niederspannung (z.B. 12 V) an die Küvettenanschlüsse und eine anschließende Strommessung (z.B. für 2 ms) bestimmt. Im Rahmen des Schritts 32 wird überprüft, ob dieser Widerstand innerhalb eines vorgegebenen Fensters liegt. Ist dies nicht der Fall, wird die Routine durch die Fehlerroutine 33 abgebrochen. Liegt der Widerstand ordnungsgemäß innerhalb des vorgegebenen Fensters, so wird die Speichereinrichtung 7 gemäß Figur 1 und 2 in Schritt 34 auf die vorgegebene Spannung U1 aufgeladen. Ist die gewünschten Ladespannung erreicht, so wird die Aufladung durch das Hochspannungsnetzteil abgeschaltet. Die Abgabe des ersten Spannungsimpulses wird dann in Schritt 35 durch Schließen des Leistungshalbleiters 9 gemäß Figur 1 und 2 ausgelöst. Dadurch entsteht ein elektrisches Feld in der Küvette, in der das zu behandelnde biologische Material vorliegt. Gleichzeitig werden in Schritt 35 die elektrischen Parameter, beispielsweise der Stromanstieg durch die Kontrolleinrichtung 20 gemäß Figur 1 und 2, sowie der zeitliche Verlauf des Spannungsimpulses durch die Steuereinheit 4 gemäß Figur 1 und 2 erfasst bzw. verfolgt. Ein zu steiler Anstieg des Stroms wird durch die Kontrolleinrichtung 20 gemäß Figur 1 und 2 erkannt und führt aus Sicherheitsgründen zu einem sofortigen Öffnen des Leistungshalbleiters 9 gemäß Figur 1 und 2 und einem Abbruch der Routine (Flankenabschaltung). Auf diese Weise kann das Auftreten einer Blitzentladung vermieden werden. In der vorliegenden Ausführungsform wird im Normalfall der erste Spannungsimpuls nach einer vorgegebenen Dauer T1, die in Schritt 36 verarbeitet wird, beendet. Hierzu wird in Schritt 37 der Leistungshalbleiter geöffnet. Wird in Schritt 36 aber festgestellt, dass die Dauer T1 aufgrund eines Abbruches des ersten Spannungsimpulses, der sich in einem Spannungsabfall manifestiert, nicht erreicht wurde, so geht die Routine zu Schritt 38 über. Hier wird nach einer voreingestellten Pausenzeit erneut Schritt 35 aufgerufen und somit eine weiterer Spannungsimpuls ausgelöst, wobei die Steuereinrichtung 4 gemäß Figur 1 und 2 die Dauer des weiteren Spannungsimpulses berechnet (T2 = T1 - Tx) und vorgibt. In Schritt 36 wird dann erneut überprüft, ob die Dauer T1 (= Tx + T2) erreicht wurde. Ist dies der Fall geht die Routine zu Schritt 37 über und beendet den Spannungsimpuls. Wird der weitere Spannungsimpuls dagegen ebenfalls abgebrochen, so geht die Routine erneut zu Schritt 38 über, so dass ein zusätzlicher weiterer Spannungsimpuls ausgelöst werden kann. Dabei ist die Anzahl der Wiederholungen, d.h. die maximale Zahl weiterer Spannungsimpulse vorgegeben. Im vorliegenden Ausführungsbeispiel können maximal drei weitere Spannungsimpulse (n = 3) ausgelöst werden, so dass in Schritt 36 die Routine nach einem erneuten Abbruch des dritten weiteren Spannungsimpulses zu Schritt 37 übergeht und somit letztendlich beendet wird.

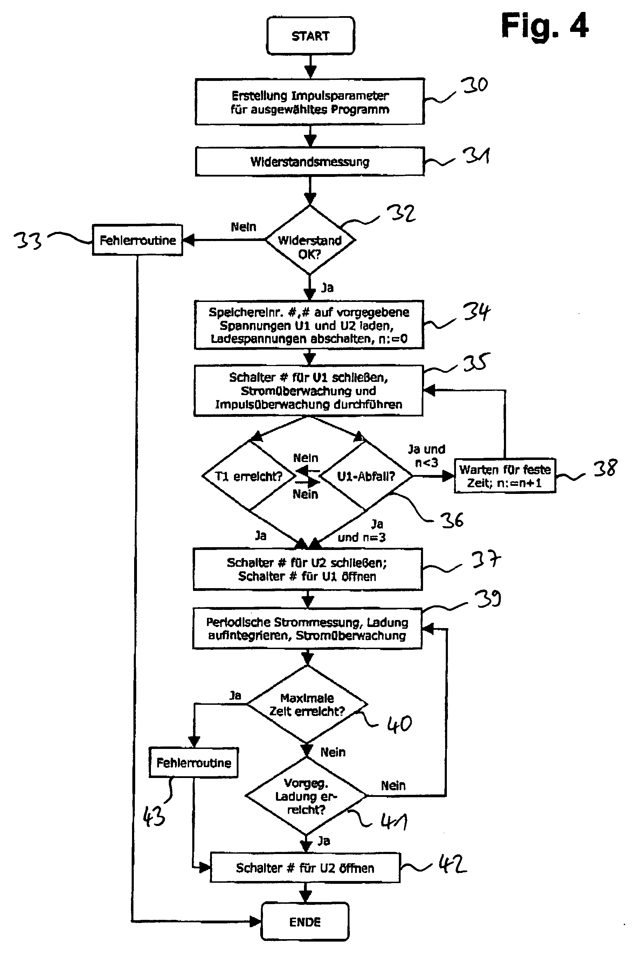

**Figur 4** zeigt ein Flussdiagramm zur Erläuterung einer besonderen Ausführungsform des erfindungsgemäßen Verfahrens, bei dem zwei ineinander übergehende Spannungsimpulse abgegeben werden. In der hier beschriebenen Ausführungsform wird ein Hochspannungsimpuls nach einer vorgegebenen Zeitdauer beendet, worauf sich unmittelbar und ohne Unterbrechung ein zweiter Impuls anschließt. Das dargestellte Verfahren entspricht also bis zum Schritt 38 im wesentlichen dem in Figur 3 beschriebenen Verfahren. Im Unterschied hierzu wird in Schritt 34 zusätzlich die zweite Speichereinrichtung 8 gemäß Figur 2 auf die vorgegebene Spannung U2 aufgeladen. Sind die gewünschten Ladespannungen erreicht, so wird die Aufladung durch das entsprechende Hochspannungsnetzteil abgeschaltet. Bei dieser besonderen Ausführungsform wird in Schritt 37 der zweite Leistungshalbleiter 10 gemäß Figur 2 bereits kurze Zeit vor Öffnen des ersten Leistungshalbleiters 9 gemäß Figur 2 geschlossen, so dass sich ein unterbrechnungsfreier Übergang zwischen den beiden Impulsen ergibt. In der kurzen Zeitspanne, in der beide Leistungshalbleiter 9,10 gleichzeitig geschlossen sind, verhindert die Diode 17 gemäß Figur 2, dass eine evtl. höhere Spannung aus der Speichereinrichtung 7 in die Speichereinrichtung 8 fließen kann. Der Leistungshalbleiter 10 bleibt anschließend solange geschlossen (sofern er nicht durch einen zu hohen Strom durch die Kontrolleinrichtung 22 geöffnet wird), bis eine vorgegebene Ladung Q durch die Küvette 24 gemäß Figur 2 geflossen ist. Dazu wird in Schritt 39 in vorgegebenen Zeitintervallen (z.B. 1 ms) der durch die Küvette fließende Strom gemessen und aufintegriert. Sobald die vorgegebene Soll-Ladung erreicht ist (Schritt 41) oder eine vorgegebene Zeit überschritten wird (Schritt 40), wird der Leistungshalbleiter 10 in Schritt 42 geöffnet und die Routine beendet. Die Kapazität der Speichereinrichtung 8 gemäß Figur 2 ist so gewählt, dass die Spannung während der Dauer des zweiten Impulses allmählich bzw. langsam abfällt. Falls aufgrund einer Störung die vorgegebene Soll-Ladung auch bei nahezu vollständig entladener Speichereinrichtung immer noch nicht erreicht ist, so wird nach Überschreiten eines entsprechend gewählten Zeitlimits der Vorgang über die Fehlerroutine 43 abgebrochen.

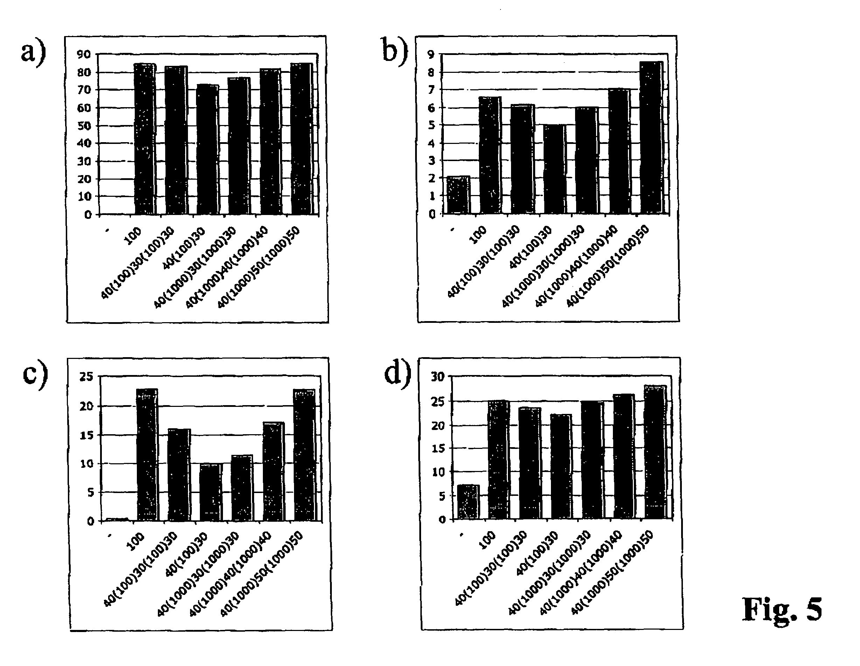

**Figur 5** zeigt Diagramme experimenteller Daten für künstlich unterbrochene Spannungsimpulse.

Zur Ermittlung der Daten für die **Figuren 5a und 5b** wurden je 1x10⁶ K563-Zellen in 100 µl Lösung des *Cell Line Nucleofector Kit R* (amaxa GmbH) aufgenommen, mit 0,5 µg pEGFP-C1 (Invitrogen) versetzt und für unterschiedliche Zeit einem Feld von 5 kV/cm ausgesetzt. Anschließend wurden die Zellen in Iscove's modified Dulbecco's medium (Invitrogen) mit 2 mM GlutaMAX (Invitrogen), 100 µg/ml Streptomycin, 100 U/ml Penicillin und 10% FCS (Sigma) aufgenommen und für 48 Stunden bei 37°C und 5% CO₂ in einem befeuchteten Kulturschrank kultiviert. Anschließend wurden die Proben mittels Durchflusszytometrie (FACSCalibur, Becton Dickinson) auf GFP-Expression und nach Propidiumiodid-Färbung auf Vitalität untersucht. Dargestellt sind die prozentualen Anteile der GFP-exprimierenden Zellen (a) und der prozentuale Anteil an toten Zellen (b). Die Beschriftung der Balken bezieht sich auf die Zeitschaltung der Feldexposition: alle Angaben sind in µs, Zahlen stehen für die Länge der Impulse, Zahlen in Klammern für dazwischenliegende Pausen.

Die **Figur 5a** zeigt, dass die Transfektionseffizenz eines vollständigen ersten Spannungsimpulses (T1 = 100 µs, Balken 1: über 80 %) durch das Fortsetzen eines nach einem Zeitraum Tx = 40 µs abgebrochenen ersten Spannungsimpulses mittels weiterer Spannungsimpulse (T2 = 30 µs und T3 = 30 µs, Balken 2: über 80 %) annähernd erreicht werden kann. Addiert sich dabei die Gesamtdauer Ts = T2 + T3 der weiteren Spannungsimpulse auf die Dauer T1 abzüglich des zwischen dem Beginn des ersten Spannungsimpulses und dem Abbrechen desselben liegenden Zeitraums Tx (Ts = T1 - Tx = 60 µs) und wird somit die vorgegebene Dauer T1 = 100 µs insgesamt erreicht, so kann die Transfektionseffizienz praktisch reproduziert werden. Wird T1 = 100 µs dagegen nicht erreicht (Tx + T2 = 70 µs, Balken 3: ca. 70 %), so kann die Transfektionseffizienz nicht vollständig erreicht werden. Bei diesen Beispielen betrug die Pausenzeit zwischen den Spannungsimpulsen jeweils 100 µs. Bei längeren Pausenzeiten (Balken 4-6: 1000 µs) ist es offenbar sinnvoll, die Gesamtdauer Ts der weiteren Impulse grösser als die Dauer T1 abzüglich des zwischen dem Beginn des ersten Spannungsimpulses und dem Abbrechen desselben liegenden Zeitraums Tx (Ts > T1 - Tx) zu wählen, um die Transfektionseffizenz des nicht abgebrochenen ersten Spannungsimpulses zu erreichen. Allerdings geht die damit verbundene längere Feldexposition der Zellen mit einer erhöhten Mortalitätsrate einher (Figur 5b).

Zur Ermittlung der Daten für die **Figuren 5c und 5d** wurden je 1x 10⁶ PBMC aus Buffy coats und 0,4 µg pEGFP-C1 (Invitrogen) in 100 µl Lösung aus dem *Human T cell Nucleofector Kit* (amaxa GmbH) aufgenommen. Die Analyse erfolgte wie oben nach 20 Stunden Inkubation und zusätzlicher Färbung mit einem anti-CD3-Antikörper mit Koppelung an Phycoerythrin. Dargestellt ist wiederum der prozentuale Anteil an transfizierten CD3⁺-T-Zellen (c) sowie der prozentuale Anteil an toten Zellen (bezogen auf Gesamtzellen) (d). Die Beschriftung der Balken bezieht sich auf die Zeitschaltung der Feldexposition: alle Angaben sind in µs, Zahlen stehen für die Länge der Impulse, Zahlen in Klammern für dazwischenliegende Pausen.

Die **Figuren 5c und 5d** bestätigen die Ergebnisse der in den Figuren 5a und 5b dargestellten Versuche, wobei hier die Transfektionseffizienzen insgesamt geringer liegen und somit grössere Schwankungen entstehen. Auch hier zeigt sich, dass ein abgebrochener erster Spannungsimpuls erfolgreich fortgesetzt werden kann. Dabei lässt sich bei längeren Pausenzeiten das Versuchsergebnis deutlich verbessern, wenn die Gesamtdauer Ts der weiteren Spannungsimpulse grösser als T1 - Tx gewählt wird.

### Bezugszeichenliste

- 1: Schaltungsanordnung
- 2: Einstellungseinheit
- 3: Steuerungseinheit
- 4: Steuereinrichtung
- 5: Hochspannungsnetzteil
- 6: Hochspannungsnetzteil
- 7: Speichereinrichtung
- 8: Speichereinrichtung
- 9: Leistungshalbleiter
- 10: Leistungshalbleiter
- 11: Potentialtrennstufe
- 12: Potentialtrennstufe
- 13: Spannungsschalter
- 14: Spannungsschalter
- 15: Küvettenanschluss
- 16: Widerstand
- 17: Diode
- 18: Küvettenanschluss
- 19: Widerstand
- 20: Kontrolleinrichtung
- 21: Schaltvorrichtung
- 22: Kontrolleinrichtung
- 23: Schalter
- 24: Küvette
- 25: Schaltungsanordnung
- 30: -
- 43: Schritte

## Patentansprüche

1. Verfahren zur Behandlung von biologischem Material mittels mindestens eines durch einen ersten Spannungsimpuls erzeugten elektrischen Feldes, wobei der erste Spannungsimpuls abgebrochen wird, wenn ein vorgegebener Grenzwert für einen elektrischen Parameter über- oder unterschritten wird, **dadurch gekennzeichnet, dass** der erste Spannungsimpuls nach dem Abbruch durch zumindest einen weiteren Spannungsimpuls fortgesetzt wird, wobei für den ersten Spannungsimpuls eine bestimmte Dauer T1 vorgegeben wird und die Dauer T2 des weiteren Spannungsimpulses zumindest gleich der Dauer T1 abzüglich des zwischen dem Beginn des ersten Spannungsimpulses und dem Abbrechen desselben liegenden Zeitraums Tx ist.

2. Verfahren nach Anspruch 1, **dadurch gekennzeichnet, dass** als T1 eine Dauer aus einem Intervall von 10 µs bis 1 ms ausgewählt wird.

3. Verfahren nach Anspruch 1 oder 2, **dadurch gekennzeichnet, dass** als Feldstärke des weiteren Spannungsimpulses die gleiche Feldstärke wie für den ersten Spannungsimpuls vorgegeben wird.

4. Verfahren nach Anspruch 3, **dadurch gekennzeichnet, dass** eine Feldstärke von 2 bis 10 KV/cm vorgegeben wird.

5. Verfahren nach einem der Ansprüche 1 bis 4, **dadurch gekennzeichnet, dass** zwischen dem Abbrechen des ersten Spannungsimpulses und der Erzeugung des weiteren Spannungsimpulses eine bestimmte Pausenzeit vorgegeben wird.

6. Verfahren nach Anspruch 5, **dadurch gekennzeichnet, dass** als Pausenzeit ein Zeitraum von mindestens 40 µs, vorzugsweise 50 bis 600 µs, insbesondere 100 µs, vorgegeben wird.

7. Verfahren nach einem der Ansprüche 1 bis 6, **dadurch gekennzeichnet, dass** insgesamt mindestens 2, vorzugsweise 2 oder 3, weitere Spannungsimpulse erzeugt werden, wenn die vorhergegangenen weiteren Spannungsimpulse abgebrochen werden.

8. Verfahren nach einem der Ansprüche 1 bis 7, **dadurch gekennzeichnet, dass** für den ersten Spannungsimpuls eine bestimmte Dauer T1 vorgegeben wird und die Gesamtdauer Ts der weiteren Spannungsimpulse zumindest gleich der Dauer T1 abzüglich des zwischen dem Beginn des ersten Spannungsimpulses und dem Abbrechen desselben liegenden Zeitraums Tx ist.

9. Schaltungsanordnung (1), insbesondere zur Behandlung von biologischem Material mittels mindestens eines durch einen ersten Spannungsimpuls erzeugten elektrischen Feldes, mit zumindest einer Speichereinrichtung (7) für elektrische Ladungsmengen zur Erzeugung zumindest eines Spannungsimpulses durch gezielte Entladung der Speichereinrichtung (7) und mindestens einer Kontrolleinrichtung (20) zur Steuerung der Entladung, mittels welcher der Spannungsimpuls bei Über- oder Unterschreiten eines vorgegebenen Grenzwertes für einen elektrischen Parameter abbrechbar ist, **dadurch gekennzeichnet, dass** zumindest eine Steuereinrichtung (4) zur Erfassung des zeitlichen Verlaufs des Spannungsimpulses vorgesehen ist, durch welche eine Fortsetzung der Entladung nach dem Abbruch steuerbar ist.

10. Schaltungsanordnung nach Anspruch 9, **dadurch gekennzeichnet, dass** die Steuereinrichtung (4) ein analoges signalverarbeitendes Bauteil, beispielsweise ein Kondensator, ist.

11. Schaltungsanordnung nach Anspruch 9, **dadurch gekennzeichnet, dass** die Steuereinrichtung (4) ein digitales signalverarbeitendes Bauteil, beispielsweise ein DSP, ist.

12. Schaltungsanordnung nach Anspruch 9, 10 oder 11, **dadurch gekennzeichnet, dass** die Steuereinrichtung (4) und/oder die Kontrolleinrichtung (20) mit einer Schaltvorrichtung (21) verbunden ist/sind.

13. Schaltungsanordnung nach Anspruch 12, **dadurch gekennzeichnet, dass** die Schaltvorrichtung (21), vorzugsweise über eine Potantialtrennstufe (11), mit einem Spannungsschalter (13) verbunden ist.

14. Schaltungsanordnung nach einem der Ansprüche 9 bis 13, **dadurch gekennzeichnet, dass** die Speichereinrichtung (7) mit einem Leistungshalbleiter (9) verbunden ist, über den die Entladung der Speichereinrichtung (7) erfolgt.

15. Schaltungsanordnung nach Anspruch 13 oder 14, **dadurch gekennzeichnet, dass** der Spannungsschalter (13) mit dem Leistungshalbleiter (9) verbunden ist.

16. Schaltungsanordnung nach einem der Ansprüche 12 bis 15, **dadurch gekennzeichnet, dass** die Schaltvorrichtung (21) durch die Kontrolleinrichtung (20) schaltbar ist.

17. Schaltungsanordnung nach einem der Ansprüche 9 bis 16, **dadurch gekennzeichnet, dass** die Kontrolleinrichtung (20) eine Abschaltvorrichtung enthält, welche die Entladung abbricht, wenn ein vorgegebener Grenzwert für einen elektrischen Parameter über- oder unterschritten wird.

18. Schaltungsanordnung nach einem der Ansprüche 12 bis 17, **dadurch gekennzeichnet, dass** die Schaltvorrichtung (21) durch die Steuereinrichtung (4), vorzugsweise über die Kontrolleinrichtung (20), schaltbar ist.

## Claims

1. Method for treating biological material by means of at least one electrical field generated by a first voltage pulse, wherein the first voltage pulse is terminated when the value for an electrical parameter goes beyond or below a predetermined threshold, **characterized in that** the first voltage pulse is continued after the termination by at least one additional voltage pulse, wherein a specific duration T1 is preset for the first voltage pulse, and the duration T2 of the additional voltage pulse is at least equal to the duration T1 minus the time Tx lying between the beginning of the first voltage pulse and the termination thereof.

2. Method according to claim 1, **characterized in that** a duration from an interval ranging from 10 µs to 1 ms is selected as T1.

3. Method according to claim 1 or 2, **characterized in that** the same field strength as for the first voltage pulse is preset as the field strength of the additional voltage pulse.

4. Method according to claim 3, **characterized in that** a field strength of 2 to 10 KV/cm is preset.

5. Method according to any one of claims 1 to 4, **characterized in that** a specific pause time is preset between the termination of the first voltage pulse and the generation of the additional voltage pulse.

6. Method according to claim 5, **characterized in that** a period of at least 40 µs, preferably 50 to 600 µs, in particular 100 µs, is preset as the pause time.

7. Method according to any one of claims 1 to 6, **characterized in that** a total of at least 2, preferably 2 or 3, additional voltage pulses are generated if the preceding additional voltage pulses are terminated.

8. Method according to any one of claims 1 to 7, **characterized in that** a specific duration T1 is preset for the first voltage pulse, and the overall duration Ts of the additional voltage pulses is at least equal to the duration T1 minus the time Tx lying between the beginning of the first voltage pulse and the termination thereof.

9. Circuit arrangement (1), in particular for treating biological material by means of at least one electrical field generated by a first voltage pulse, comprising at least one storage device (7) for electrical charges for generating at least one voltage pulse by selectively discharging the storage device (7), and at least one control unit (20) for controlling the discharge, by means of which the voltage pulse can be terminated when the value for an electrical parameter goes beyond or below a predetermined threshold, **characterized in that** at least one controller (4) for monitoring the chronological progression of the voltage pulse is provided, by means of which a continuation of the discharge after termination can be controlled.

10. Circuit arrangement according to claim 9, **characterized in that** the controller (4) is an analog signal-processing module, for example, a capacitor.

11. Circuit arrangement according to claim 9, **characterized in that** the controller (4) is a digital signal-processing module, for example, a DSP.

12. Circuit arrangement according to claim 9, 10, or 11, **characterized in that** the controller (4) and/or the control unit (20) is/are connected to a switching device (21).

13. Circuit arrangement according to claim 12, **characterized in that** the switching device (21) is connected to a voltage switch (13), preferably via a separator stage (11).

14. Circuit arrangement according to any one of claims 9 to 13, **characterized in that** the storage device (7) is connected to a power semiconductor (9), via which the storage device (7) is discharged.

15. Circuit arrangement according to claim 13 or 14, **characterized in that** the voltage switch (13) is connected to the power semiconductor (9).

16. Circuit arrangement according to any one of claims 12 to 15, **characterized in that** the switching device (21) can be operated by the control unit (20).

17. Circuit arrangement according to any one of claims 9 to 16, **characterized in that** the control unit (20) contains a disconnecting device that terminates the discharge when the value for an electrical parameter goes beyond or below a predetermined threshold.

18. Circuit arrangement according to any one of claims 12 to 17, **characterized in that** the switching device (21) can be operated by the controller (4), preferably via the control unit (20).

## Revendications

1. Procédé pour le traitement d'un matériau biologique à l'aide d'au moins un champs électrique généré par une première impulsion de tension, la première impulsion de tension étant interrompue lorsqu'une valeur limite prescrite pour un paramètre électrique est dépassée par le haut ou par le bas, **caractérisé en ce que** la première impulsion de tension, après l'interruption, se poursuit grâce à au moins une autre impulsion de tension, une durée T1 déterminée étant prescrite pour la première impulsion de tension et la durée T2 de l'autre impulsion de tension étant au moins égale à la durée T1 après déduction du laps de temps Tx se situant entre le début de la première impulsion de tension et l'interruption de cette même.

2. Procédé selon la revendication 1, **caractérisé en ce que** l'on choisit, en tant que T1, une durée dans un intervalle de 10 µs à 1 ms.

3. Procédé selon les revendications 1 ou 2, **caractérisé en ce que**, en guise puissance de champ de l'autre impulsion de tension, on prescrit la même puissance de champ que pour la première impulsion de tension.

4. Procédé selon la revendication 3, **caractérisé en ce qu'**une puissance de champ de 2 à 10 KV/cm est prescrite.

5. Procédé selon l'une des revendications 1 à 4, **caractérisé en ce que** l'on prescrit un temps de pause déterminé entre l'interruption de la première impulsion de tension et la génération de l'autre impulsion de tension.

6. Procédé selon la revendication 5, **caractérisé en ce que** l'on prescrit, en guise de temps de pause, un laps de temps d'au moins 40 µs, de préférence de 50 à 600 µs, en particulier de 100 µs.

7. Procédé selon l'une des revendications 1 à 6, **caractérisé en ce que** l'on génère globalement au moins 2, de préférence 2 ou 3, autres impulsions de tension lorsque les autres impulsions de tension précédentes sont interrompues.

8. Procédé selon l'une des revendications 1 à 7, **caractérisé en ce qu'**une durée T1 déterminée est prescrite pour la première impulsion de tension et **en ce que** la durée globale Ts des autres impulsions de tension est au moins égale à la durée T1 après déduction du laps de temps Tx entre le début de la première impulsion de tension et l'interruption de cette même.

9. Dispositif de circuits (1), en particulier pour le traitement d'un matériau biologique à l'aide d'au moins un champs électrique généré par une première impulsion de tension, avec au moins un dispositif d'accumulation (7) pour des quantités de charge électrique afin de générer au moins une impulsion de tension grâce à une décharge ciblée du dispositif d'accumulation (7) et au moins un dispositif de contrôle (20) pour la commande de la décharge, grâce auquel l'impulsion de tension peut être interrompue en cas de dépassement par le haut ou par le bas d'une valeur limite prescrite pour un paramètre électrique, **caractérisé en ce que** l'on prévoit au moins un dispositif de commande (4) pour l'acquisition de la variation dans le temps de l'impulsion de tension, grâce auquel il est possible de commander une reprise de la décharge après l'interruption.

10. Dispositif de circuits selon la revendication 9, **caractérisé en ce que** le dispositif de commande (4) est un composant de traitement de signal analogique, par exemple un condensateur.

11. Dispositif de circuits selon la revendication 9, **caractérisé en ce que** le dispositif de commande (4) est un composant de traitement de signal numérique, par exemple un DSP.

12. Dispositif de circuits selon les revendications 9, 10 ou 11, **caractérisé en ce que** le dispositif de commande (4) et/ou le dispositif de contrôle (20) est/sont en liaison avec un dispositif de commutation (21).

13. Dispositif de circuits selon la revendication 12, **caractérisé en ce que** le dispositif de commutation (21) est en liaison avec commutateur de tension (13), de préférence par l'intermédiaire d'un étage de séparation de potentiel (11).

14. Dispositif de circuits selon l'une des revendications 9 à 13, **caractérisé en ce que** le dispositif d'accumulation (7) est en liaison avec un semiconducteur de puissance (9) par l'intermédiaire duquel s'effectue la décharge du dispositif d'accumulation (7).

15. Dispositif de circuits selon les revendications 13 ou 14, **caractérisé en ce que** le commutateur de tension (13) est en liaison avec le semi-conducteur de puissance (9).

16. Dispositif de circuits selon l'une des revendications 12 à 15, **caractérisé en ce que** le dispositif de commutation (21) peut être commuté grâce au dispositif de contrôle (20).

17. Dispositif de circuits selon l'une des revendications 9 à 16, **caractérisé en ce que** le dispositif de contrôle (20) contient un dispositif d'arrêt, lequel interrompt la décharge lorsqu'une valeur limite prescrite pour un paramètre électrique est dépassée par le haut ou par le bas.

18. Dispositif de circuits selon l'une des revendications 12 à 17, **caractérisé en ce que** le dispositif de commutation (21) peut être commuté grâce au dispositif de commande (4), de préférence via le dispositif de contrôle (20).
